# EUROPEAN PATENT SPECIFICATION

(11) **EP 2 349 404 B1**
(45) Date of publication and mention of the grant of the patent: **13.11.2013**
(21) Application number: 09782879.2
(22) Date of filing: 10.09.2009
(51) Int. Cl.: A61M 5/162, A61M 39/10

(54) **PIERCING DEVICE FOR BAGS CONTAINING MEDICAL FLUIDS**
VORRICHTUNG ZUM DURCHSTECHEN VON MEDIZINISCHE FLÜSSIGKEITEN BEINHALTENDEN BEUTELN
DISPOSITIF DE PERÇAGE POUR POCHES CONTENANT DES FLUIDES MÉDICAUX

(30) Priority: 11.09.2008 IT MO20080226
(43) Date of publication of application: 03.08.2011
(73) Proprietor: ARIES S.r.l., 41037 Mirandola (Modena) (IT)
(72) Inventor: SOFFRITTI, Massimo, I-41037 Mirandola (IT)
(74) Representative: Reniero, Cirillo Silvano
(86) International application number: PCT/EP2009/061763
(87) International publication number: WO 2010/029129

(56) References cited:
- DE-A1-102005 015 504
- US-A- 5 071 413
- US-B1- 6 485 472

## Description

### Technical field

The present invention relates to a piercing device, particularly for bags for containing physiological fluids for medical use and the like.

### Background Art

As is known, bags containing medical fluids, such as for example physiological solutions or others, are increasingly widespread in the hospital sector. These bags can be used for the administration of drugs, which are added to the main content of the bag itself.

Within hospital facilities there are rooms assigned to the preparation of drugs, which are then transported to the wards, by means of such bag, for administration to the patients.

A substantial part of these preparations is assigned to the mixing of antiblastic drugs.

Currently, tumor treatment provides for the use of cytotoxic substances, which are administered to the patient predominantly intravenously. The toxicity of these substances makes them particularly dangerous for operators who handle them and come into contact with them. These drugs are often supplied by pharmaceutical companies in concentrated form (typically contained in bottles, vials or bags) and must be diluted in suitable solvents (saline solution or glucose solution) in order to be infused to patients. Therefore, the first maneuver that must be performed is withdrawal of the drug and dilution, typically in a bag with the cited solvents. The operation is performed by means of piercing devices, which are inserted in one of the access paths of the bag.

Piercing devices, particularly for bags for containing physiological fluids for medical use and the like, are known which comprise at least one substantially tubular and internally hollow body that forms a through cavity between a first end and a second end of such body.

In particular, the first end is provided with a bag piercing tip.

The second end, which is arranged opposite the first end, can be associated with a device for the infusion or administration of the physiological fluid contained in the bag and/or with means for temporary closure of the cavity, such as syringes, caps, and the like.

In view of the dangerousness/toxicity of the drugs and their high cost, it appears therefore indispensable to have a safe access for injecting the drug into the bag, in order to avoid spillage thereof and possible contaminations.

The diluted and prepared drug is then transported from the ward where it is prepared to the wards where it is to be administered.

As can be deduced easily, the step of transporting the bag, pierced earlier and containing the prepared drug, is another critical moment due to possible contamination of the operators.

The drug is then administered to the patients by means of dedicated infusion lines.

The administration step also is critical in terms of drug leaks, because during this operation the patient may be able to walk and therefore move, for example, with an IV stand or the like.

These known types of piercing device in any case are not free from drawbacks, which include the fact that during their use they are subject to accidental separations from the bag.

Any accidental pulling of the infusion line while the patient is walking or of the medical staff during transport or others may in fact cause separations of the piercing device from the bag, leading to the above cited problems.

Separation of the piercing device from the bag, with consequent outflow of drug, places at risk the operator, the patients and the administration spaces and triggers delicate procedures for cleaning and decontaminating these environments, including considerable inconvenience for the hospital environment.

Moreover, as is known, antiblastic drugs are very expensive and accordingly even minimal residual quantities, if they remained within the bag, in addition to those that can be lost due to the accidental events described above, become relevant in terms of economic management of such drug.

All known piercing devices of the known type have piercing tips of considerable length, which prevent complete outflow of the medical fluids and therefore complete emptying of the bag, with consequent waste of the drug. US-5 071 413 teaches a device which can be anchored by snap-fitting or bayonet means to a respective connection element fixed to a liquid containment bag.

### Disclosure of the Invention

The aim of the present invention is to eliminate the above-mentioned drawbacks of the background art, by providing a piercing device, particularly for bags for containing physiological fluids for medical use and the like, that allows safe, stable and quick connection to the bag for containing the medical fluid and allows to eliminate the danger of accidental leaks of pharmacological fluid or accidental separations between the piercing device and the bag.

Within this aim, an object of the present invention is to reduce, if not eliminate completely as much as possible, wastes of medical fluid both during the steps of preparation for the administration of said fluid and in the transport and administration steps themselves.

Another object of the piercing device according to the invention is to ensure the safety of the personnel assigned to administration of the drug and of the patient to whom the drug is administered, as well as the healthiness of the environment for preparation, transport and administration of said pharmacological fluid.

Another object of the invention is to facilitate complete emptying of the pharmacological fluid from the containment bag, thus reducing wastes of pharmacological material.

Another object of the present invention is to provide a device that is simple, relatively easy to provide in practice, safe in use, even in operating conditions in tight maneuvering spaces, such as for example for operations under a hood, and effective in operation as well as of relatively low cost.

This aim and these and other objects which will become better apparent hereinafter are achieved by a piercing device according to claim 1.

### Brief description of the drawings

Further characteristics and advantages of the present invention will become better apparent from the following detailed description of a preferred but not exclusive embodiment of a piercing device, particularly for bags for containing physiological fluids for medical use and the like, illustrated by way of non-limiting example in the accompanying drawings, wherein:

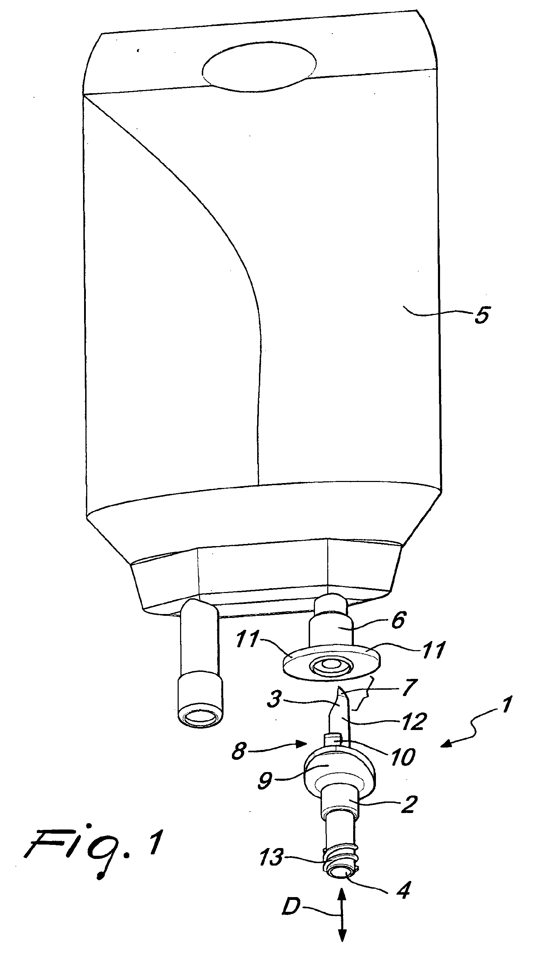

Figure 1 is a perspective view of a piercing device according to the invention and of a bag for containing a medical fluid;

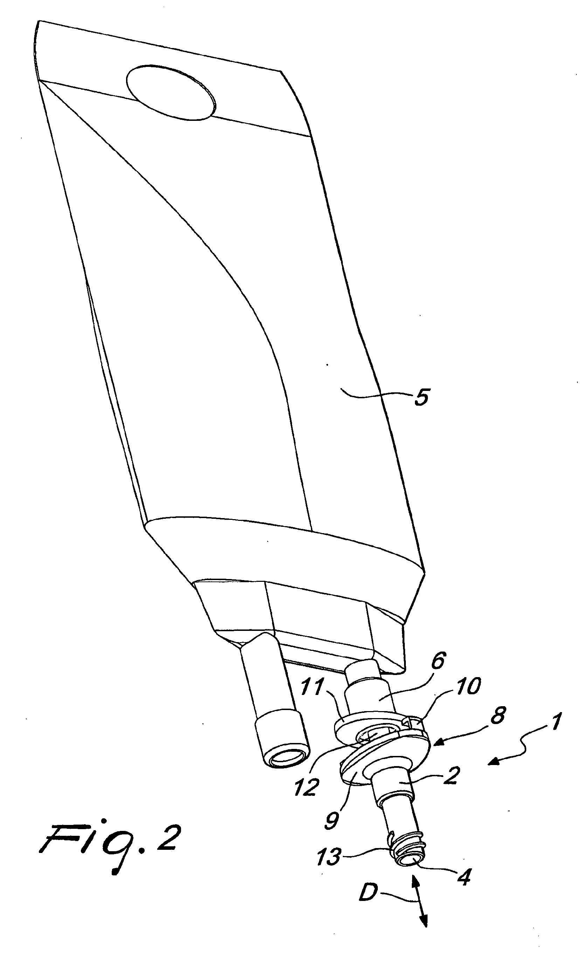

Figure 2 is a perspective view of the piercing device according to the invention, partially inserted in an access element of the containment bag;

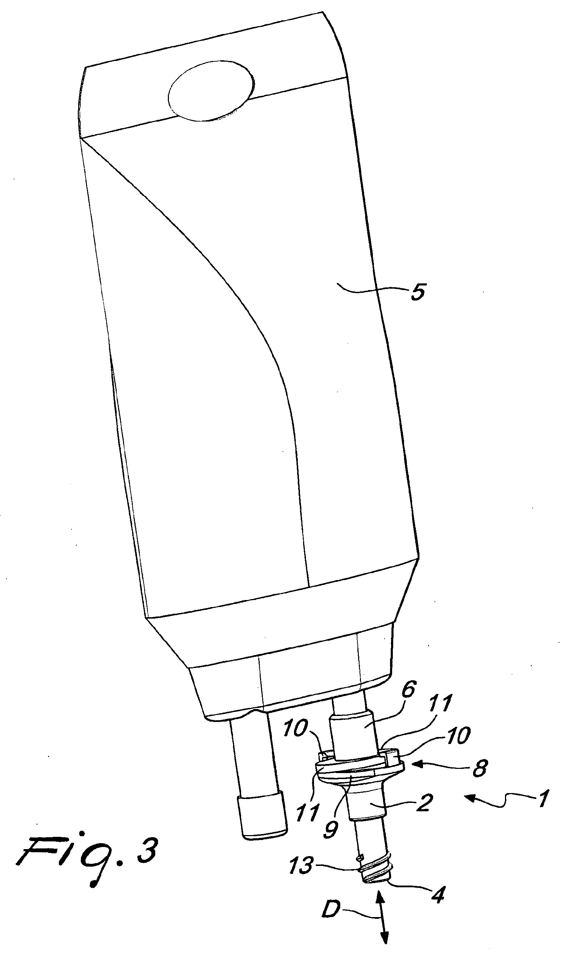

Figure 3 is a perspective view of a piercing device according to the invention, inserted in an access element of the containment bag;

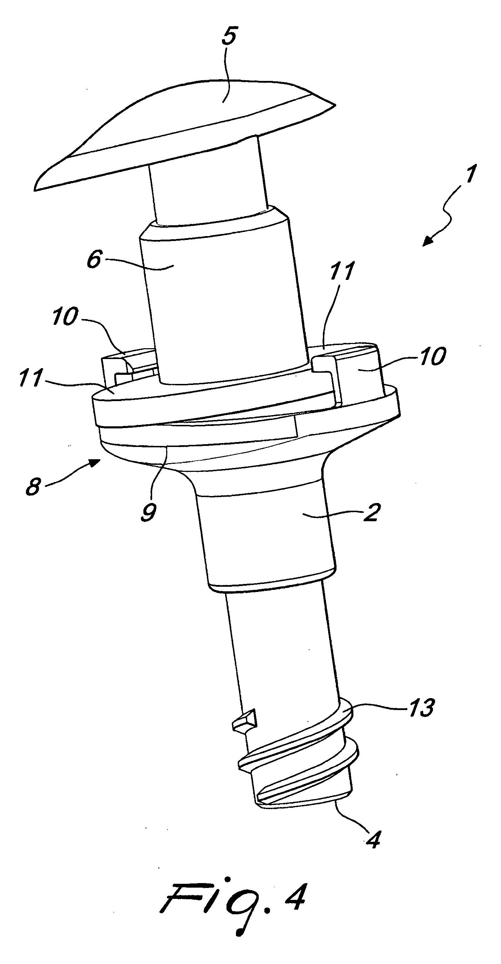

Figure 4 is a view of a detail of the device of Figure 3;

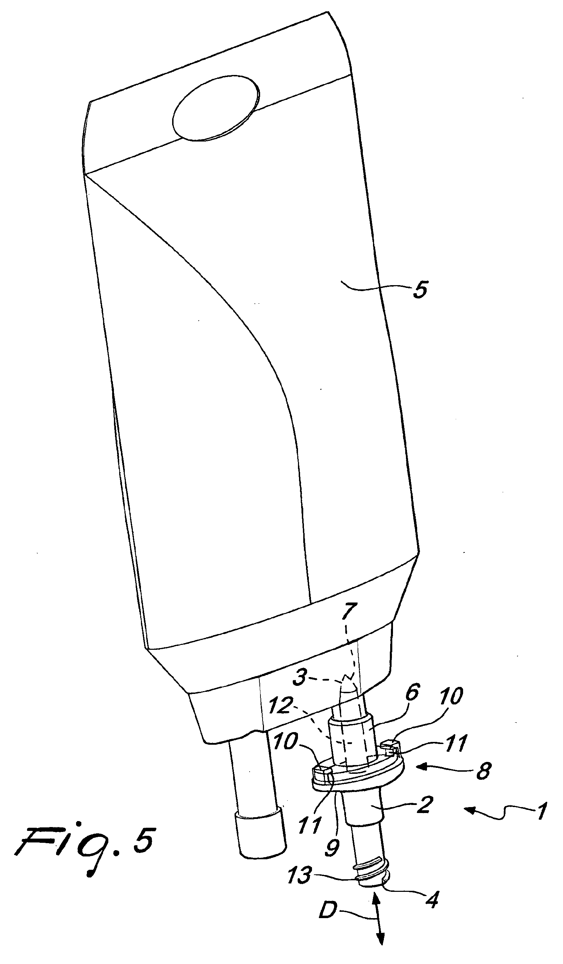

Figure 5 is a perspective view of the piercing device according to the invention, inserted in the access element and retained thereto;

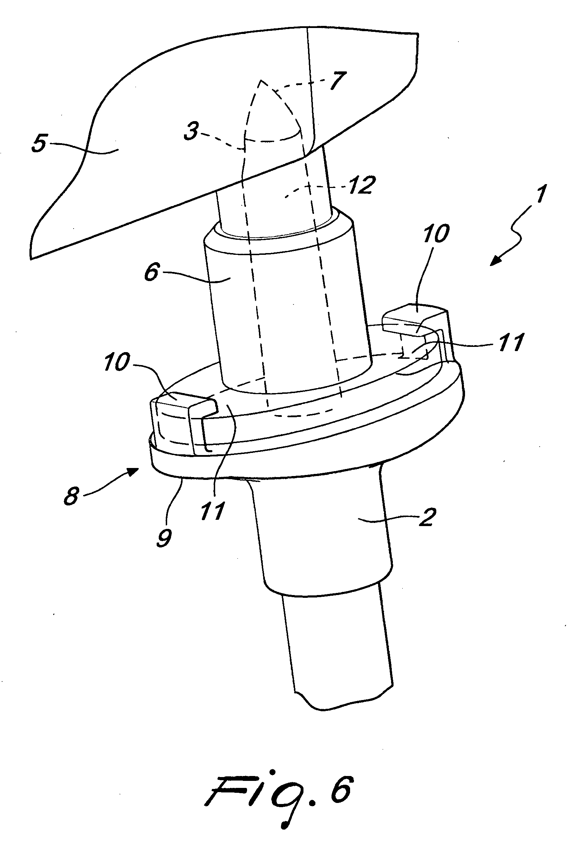

Figure 6 is a view of a detail of the device of Figure 5;

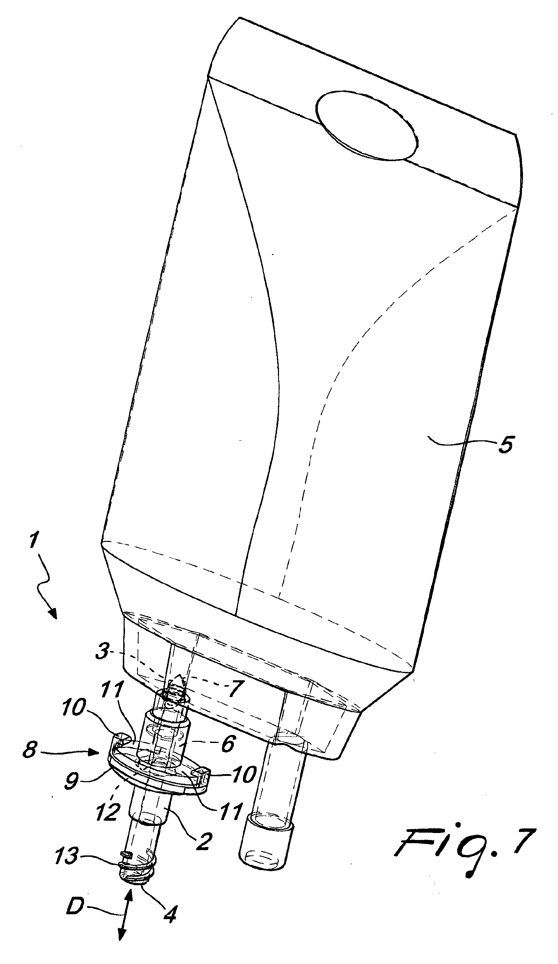

Figure 7 is a rear perspective view of Figure 5;

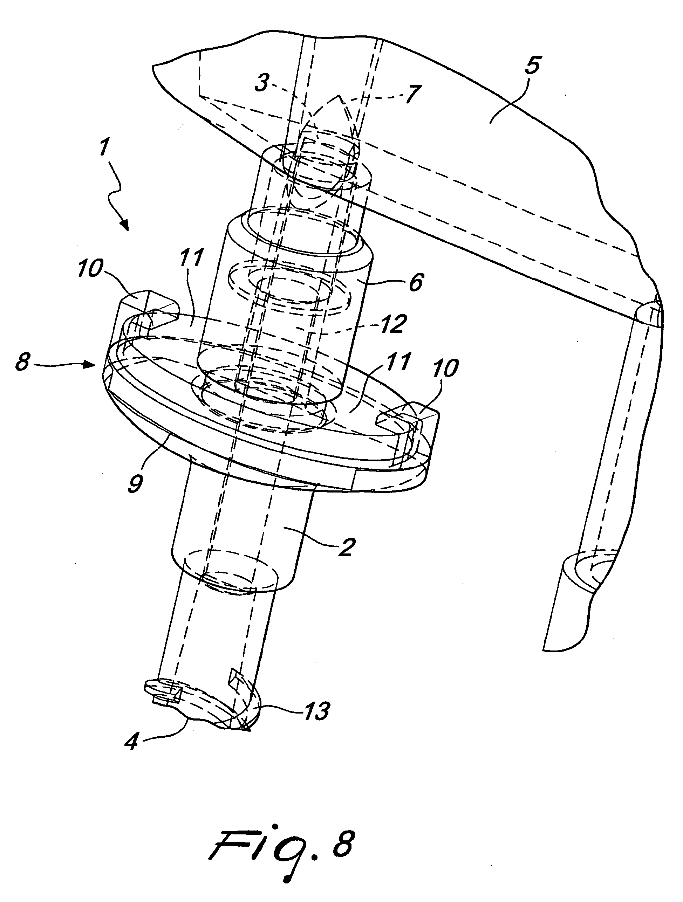

Figure 8 is a rear perspective view of Figure 6.

### Ways of carrying out the Invention

With reference to the figures, the reference numeral 1 generally designates a piercing device, particularly for bags for containing physiological fluids for medical use and the like.

The device 1 comprises at least one substantially tubular body 2 which is internally hollow and is open at its ends 3, 4.

The tubular body 2, in particular, can be inserted along a direction D for the mating of the tubular body 2 and a containment bag 5, in the direction for their mutual approach, in at least one access element 6 of said containment bag.

The device 1 further comprises at least one tip 7 for piercing the access element 6, which is associated with a first end 3 of the tubular body 2.

The second end 4 of said tubular body, which lies opposite the first end, can be associated with a device for the infusion or administration of the physiological fluid contained in the containment bag 5, which for example is of the type of an infusion line, a syringe or other technically equivalent element, and/or with means for temporarily closing the second end 4, for example a cap, a syringe or valve means and the like, which are not shown in the figures since they are of a type that is known to the person skilled in the art.

In particular, the device 1 comprises means 8 for removable retention along the direction D for mating the tubular body 2 to at least one between said containment bag and/or the access element 6, in the direction of mutual spacing.

The device 1 comprises at least one flange-like body 9, which is jointly associated with the tubular body 2.

The flange-like body 9 is advantageously formed in a region that is intermediate between the first end 3 and the second end 4; the flange-like body 9 is in fact adapted to abut against the access element 6.

The retention means 8, in particular, are associated with the flange-like body 9 for removable retention of the access element 6 coupled to each other.

The retention means 8 advantageously comprise at least one hook 10 for retention of the access element 6 along the mating direction D, in the direction for mutual spacing of the device 1 from the containment bag 5.

The retention means 8, in the preferred embodiment shown in the figures, comprise at least one pair of hooks 10, which are associated with the flange-like body 9.

The retention means 8 and the hooks 10 are adapted to interfere with an engagement region 11 that is formed externally with respect to the access element 6, for the retention of a portion 12 of the tubular body 2, which is comprised between the tip 7 and the flange-like body 9, within the access element 6.

In particular, the engagement region 11 is formed in the perimetric edge of the access element 6 that lies furthest from the containment bag 5.

In the preferred embodiment shown in the figures, the perimetric edge of the access element 6 that lies furthest from the containment bag 5, has a substantially flange-like and substantially elliptical shape; the engagement region 11 is formed on the facing surface of the perimetric edge of the access element 6 that faces the containment bag 5 and advantageously on the major axis of this ellipse.

The access element 6 is generally of the type of a substantially cylindrical hollow body, whose end that lies closest to the containment bag 5 is blocked by a membrane, as is known to the person skilled in the art, which can be torn by the tip 7 of the device 1 and keeps said containment bag closed until the device 1 is inserted in said access element.

In particular, the portion 12 of the tubular body 2 is further suitable for insertion by sliding snugly within at least one portion of the access element 6 to pierce the containment bag 5 and therefore the tearable membrane.

In particular, the portion 12 has a substantially cylindrical or conical hollow shape, the radius of the outer surface of at least one part of said portion is substantially equal to the radius of the internal surface of at least one part of the access element 6, so as to provide a snug sliding connection and thus prevent the fluid contained in the containment bag 5 from being able to flow out of it (laterally with respect to the device 1) once the membrane has been torn.

In particular, the rotation, with respect to the longitudinal axis of the portion 12, of the flange-like body 9 with respect to the access element 6, which occurs after the translational motion of the device 1 along the mating direction D in the direction for approach to the containment bag 5 and causes the flange-like body 9 to abut against the access element 6, moves the retention means 8 and therefore the hooks 10 so as to interfere with the engagement region 11 for retention, along the mating direction D, in the direction of mutual spacing, of the portion 12 (of the tubular body 2) within the access element 6.

In an alternative embodiment, not shown in the figures, the retention means 8, and therefore the hooks 10, can be made of elastically yielding material for the interlocking engagement of the tubular body 2 and/or of the flange-like body 9, in any case of the device 1, with the access element 6.

Advantageously, the portion 12 has a length that is at least substantially equal to the length of the access element 6.

The tip 7, during the piercing of the access element 6, is adapted to protrude at least partially into the containment bag 5 proximate to the access element.

This configuration of the portion 12 and of the tip 7 ensures in particular the complete emptying of the containment bag 5.

Moreover, the tubular body 2, the flange-like body 9 and the retention means 8 are provided as a single part so as to form a monolithic body.

For example, the device 1 is provided by injection-molding in a mold of a suitable shape, by injection of medical-grade plastic material, such as for example acrylonitrile-butadiene-styrene (ABS), polyvinyl chloride (PVC) or polycarbonate (PC), which ensures its excellent ergonomics and breaking strength.

Advantageously, the second end has at least one connector 13 of the Luer-Lok type, which according to the constructive requirements can be of the male or female type.

This Luer-Lok connector 13 makes the device 1 particularly suitable to be associated safely with any infusion device for medical use and any instrument provided with a Luer-Lok connector that is complementary thereto.

By way of example, the device 1 can be associated with syringes, one-way or two-way valves, glued to tubes to form sets for administration or other known medical devices.

Advantageously, the containment bag 5 and the piercing device 1, together with the optional infusion or administration device or means for temporary closure of the second end 4 which can be associated with the piercing device 1, can be offered in a kit for the transport and administration of physiological fluids for medical use and the like.

The operation of the device according to the present invention is as follows.

In order to pierce the membrane for temporary closure of the containment bag 5 it is sufficient to insert the portion 12 and therefore the tip 7 in the access element 6.

The sliding, along the mating direction D in the direction for mutual approach of the device 1 to the containment bag 5, of the portion 12 inside said access element makes the tip 7 tear said membrane, opening the containment bag 5.

By turning the device 1 with respect to its own longitudinal axis, once the flange-like body 9 is arranged in abutment against the access element 6, the hooks 10 move so as to interfere with the engagement region 11, retaining the device 1 to the access element 6, which are mutually jointly connected.

In this work configuration, the assigned operator, having previously connected the second end 4 of the device 1 to an element for temporary closure, for example a syringe or a valve or a cap, can act without risks of contamination, for example for operations for diluting the drug, drawing it or others.

Moreover, the operator can carry the containment bag 5 in full safety.

Even in conditions of use of the containment bag 5, in which it is connected to the patient by means of the administration line, for example fastened to movable stands or the like, the device 1, retained jointly to the access element 6 by the retention means 8, prevents the danger of accidental leakages of drug from the containment bag 5 or accidental separations between said device and said containment bag.

In practice it has been found that the described invention achieves the proposed aim and objects, and in particular the fact is stressed that the piercing device according to the invention is capable of connecting jointly to ordinary bags that contain physiological solution. The piercing device therefore can be associated with the access element that is present in the lower part of the bag, can tear the protective membrane that is present in said access element and can be fixed so that it cannot be detached/extracted accidentally. Once the piercing element has been inserted, it constitutes a safe path for access to the content of the bag or allows to perform additions of drugs or solutions within said bag.

Moreover, in view of the particular shape of the piercing tip, the device according to the invention allows complete emptying of the bag, with consequent recovery of all the pharmacological content.

The invention thus conceived is susceptible of numerous modifications and variations.

All the details may further be replaced with other technically equivalent elements.

In practice, the materials used, as well as the contingent shapes and dimensions, may be any according to requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A piercing device (1), particularly for bags (5) for containing physiological fluids for medical use and the like, comprising at least one substantially tubular and internally hollow body (2) that is open at its ends (3, 4), said tubular body (2) being insertable along a mating direction (D), in the direction of approach toward a containment bag (5), in at least one access element (6) of said containment bag (5), the access element (6) comprising a peripheral edge that lies furthest from said containment bag (5), which is substantially flange-like and substantially elliptical in shape, at least one tip (7) for piercing said access element (6) associated with a first one (3) of said ends, the second one (4) of said ends, which lies opposite said first end (3), being associable with a device for the infusion or administration of the physiological fluid contained in the containment bag and/or with means for temporary closure of said second end (4), said piercing device further comprising means (8) for the removable retention, along said mating direction (D), of said tubular body (2) to at least one between said containment bag (5) and/or said access element (6), in the direction for mutual spacing, said device comprising at least one flange-like body (9) jointly associated with said tubular body (2) and advantageously formed in an intermediate region between said first end (3) and said second end (4); said flange-like body (9) being suitable for abutting against said access element (6), and said retention means (8) being associated with said flange-like body (9) for removably retaining said access element (6) and the flange-like body (9) coupled together, **characterized In that** said retention means (8) comprises at least one pair of hooks associated with the flange-like body and **in that** said hooks are spaced from one another for a distance greater than the smaller axis of said elliptical perimeter edge, said distance substantially corresponding to the major axis of said elliptical perimetric edge, whereby, after the translational motion of said device (1) along said mating direction (D) in the approaching direction to said containment bag (5) for causing said flange-like body (9) to abut against said access element (6) by rotating said flange-like body (9) with respect to said access element (6), the hooks (10) are brought to interfere with an engagement region (11) of said access element (6) in order to retain, along the mating direction (D) In the mutual spacing direction, a portion (12) of said tubular body (2) within said access element (6).

2. The device (1) according to claim 1, **characterized in that** said portion (12) is suitable for insertion by sliding snugly within at least one portion of said access element (6) for the piercing of said containment bag (5).

3. The device (1) according to claim 1 or 2, **characterized in that** said hooks (10) are made of elastically yielding material for the interlocking coupling of said tubular body (2) and/or said flange-like body (9) to said access element (6).

4. The device (1) according to one or more of the preceding claims, **characterized in that** said portion (12) has a length that is at least substantially equal to the length of said access element (6), said tip (7), during piercing, being adapted to protrude at least partially in said containment bag (5) proximate to the access element (6).

5. The device (1) according to one or more of the preceding claims, **characterized in that** said tubular body (2), said flange-like body (9) and said retention means (8) are provided as a single part so as to form a monolithic body.

6. The device (1) according to one or more of the preceding claims, **characterized in that** said second end (4) is provided with at least one connector of the Luer-Lok type.

7. A kit for transport and administration of physiological fluids for medical use and the like, comprising at least one bag (5) for the containment of at least one physiological fluid for medical use provided with at least one access element (6), the access element (6) comprising a perimetric edge that lies furthest from said containment bag (5), which is substantially flange-like and substantially elliptical in shape, and at least one piercing device (1) according to one or more of claims 1 to 6.

8. The kit according to claim 7, further comprising at least one between an infusion or administration device and means for temporary closure of said second end (4) which can be associated with said piercing device (1).

## Patentansprüche

1. Eine Durchstechvorrichtung (1), insbesondere für Beutel (5) zum Beinhalten von physiologischen Fluiden zur medizinischen Verwendung und Ähnliches, die zumindest einen im Wesentlichen röhrenförmigen und innen hohlen Körper (2) aufweist, der an seinen Enden (3, 4) offen ist, wobei der röhrenförmige Körper (2) entlang einer Passrichtung (D) in Richtung einer Annäherung in Richtung eines Beinhaltungsbeutels (5) einfügbar ist in zumindest ein Zugangselement (6) des Beinhaltungsbeutels (5), wobei das Zugangselement (6) einen peripheren Rand aufweist, der am weitesten von dem Beinhaltungsbeutel (5) weg liegt, welcher im Wesentlichen flanschähnlich und im Wesentlichen von elliptischer Form ist, zumindest eine Spitze (7) zum Durchstechen des Zugangselements (6), welches einem ersten (3) der Enden und dem zweiten (4) der Enden zugeordnet ist, wobei das zweite (4) gegenüber dem ersten Ende (3) liegt, und einer Vorrichtung für die Infusion oder Verabreichung des physiologischen Fluides zuordenbar ist, welches in dem Beinhaltungsbeutel beinhaltet ist und/oder mit Mitteln zum zeitweisen Verschließen des zweiten Endes (4) zuordenbar ist, wobei die Aufstechvorrichtung weiterhin Mittel (8) aufweist für das entfernbare Zurückhalten des röhrenförmigen Körpers (2) entlang der Passrichtung (D) in zumindest entweder dem Zurückhaltungsbeutel (5) und/oder dem Zugangselement (6) in der Richtung einer wechselseitigen Beabstandung,
wobei die Vorrichtung zumindest einen flanschähnlichen Körper (9) aufweist, der dem röhrenförmigen Körper (2) verbindbar zugeordnet ist, und in einem Zwischenbereich zwischen dem ersten Ende (3) und dem zweiten Ende (4) vorteilhaft geformt ist; wobei der flanschähnliche Körper (9) zum Anstoßen gegen das Zugangselement (6) geeignet ist, und das Rückhaltemittel (8) dem flanschähnlichen Körper (9) zum entfernbaren Zurückhalten des Zugangselements (6) und des flanschähnlichen Körpers (9) zugeordnet ist, welche miteinander gekoppelt sind,
**dadurch gekennzeichnet, dass**
das Rückhaltemittel (8) zumindest ein Paar Haken aufweist, die dem flanschähnlichen Körper zugeordnet sind, und dadurch, dass die Haken voneinander mit einem Abstand beabstandet sind, der größer als die kleinere Achse des elliptischen Umfangsrandes ist, wobei der Abstand im Wesentlichen der Hauptachse des elliptischen Umfangsrandes entspricht, wobei nach der translatorischen Bewegung der Vorrichtung (1) entlang der Passrichtung (D) in der Annäherungsrichtung zu dem Beinhaltungsbeutel (5) zum Verursachen, dass der flanschähnliche Körper (9) gegen das Zugangselement (6) stößt durch Drehen des flanschähnlichen Körpers (9) relativ zu dem Zugangselement (6) die Haken (10) in Interaktion mit einem Einwirkungsbereich (11) des Zugangselements (6) gebracht werden, um entlang der Passrichtung (D) in der wechselseitigen Beabstandungsrichtung einen Abschnitt (12) des röhrenförmigen Körpers (2) innerhalb des Zugangselements (6) zurückzuhalten.

2. Vorrichtung (1) nach Anspruch 1, **dadurch gekennzeichnet, dass** der Abschnitt (12) zum Einfügen durch komfortables Rutschen innerhalb zumindest eines Abschnitts des Zugangselements (6) für das Durchstechen des Beinhaltungsbeutels (5) geeignet ist.

3. Vorrichtung (1) nach Anspruch 1 oder 2, **dadurch gekennzeichnet, dass** die Haken (10) aus einem elastisch nachgebenden Material für das miteinander verbindende Koppeln des röhrenförmigen Körpers (2) und/oder des flanschähnlichen Körpers (9) mit dem Zugangselement (6) hergestellt sind.

4. Vorrichtung (1) nach irgendeinem der vorhergehenden Ansprüche, **dadurch gekennzeichnet, dass** der Abschnitt (12) eine Länge aufweist, welche zumindest im Wesentlichen gleich der Länge des Zugangselements (6) ist, wobei die Spitze (7) während eines Durchstechens angepasst ist, um zumindest teilweise in den Beinhaltungsbeutel (5) nahe dem Zugangselement (6) hervorzustehen.

5. Vorrichtung (1) nach irgendeinem der vorhergehenden Ansprüche, **dadurch gekennzeichnet, dass** der röhrenförmige Körper (2), der flanschähnliche Körper (9) und die Rückhaltemittel (8) als ein einziges Teil vorgesehen sind, um einen monolithischen Körper zu bilden.

6. Vorrichtung (1) nach irgendeinem der vorhergehenden Ansprüche, **dadurch gekennzeichnet, dass** das zweite Ende (4) mit zumindest einem Stecker des Luer-Lock-Typs versehen ist.

7. Eine Ausrüstung zum Transportieren und Verabreichen von physiologischem Fluid zur medizinischen Verwendung und Ähnlichem, zumindest einen Beutel zum Beinhalten zumindest eines physiologischen Fluides zur medizinischen Verwendung aufweisend, welcher mit zumindest einem Zugangselement (6) versehen ist, wobei das Zugangselement (6) einen Umfangsrand aufweist, der am weitesten von dem Beinhaltungsbeutel (5) weg liegt, welcher im Wesentlichen flanschförmig und im Wesentlich von elliptischer Form ist, und mit zumindest einer Durchstechvorrichtung (1) gemäß einem oder mehreren der Ansprüche 1 bis 6 versehen ist.

8. Ausrüstung nach Anspruch 7, weiterhin aufweisend zumindest entweder eine Infusions- oder Verabreichungsvorrichtung und Mittel zum temporären Verschließen des zweiten Endes (4), welches der Durchstechvorrichtung (1) zugeordnet werden kann.

## Revendications

1. Un dispositif de perçage (1), en particulier pour des poches (5) destinées à contenir des fluides physiologiques pour usage médical et analogues, comprenant au moins un corps substantiellement tubulaire et intérieurement creux (2) qui est ouvert à ses extrémités (3, 4), ledit corps tubulaire (2) pouvant être inséré le long d'une direction de conjugaison (D), dans la direction d'approche en direction d'une poche de rétention (5), dans au moins un élément d'accès (6) de ladite poche de rétention (5), l'élément d'accès (6) comprenant un rebord périphérique qui s'étend plus loin que ladite poche de rétention (5), qui est substantiellement semblable à une collerette et de forme substantiellement elliptique, au moins un embout (7) pour percer ledit élément d'accès (6) associé à une première (3) desdites extrémités, la seconde (4) desdites extrémités, qui s'étend à l'opposé de ladite première extrémité (3), pouvant être associée à un dispositif pour la perfusion ou l'administration du fluide physiologique contenu dans la poche de rétention et/ou à des moyens de fermeture temporaire de ladite seconde extrémité (4), ledit dispositif de perçage comprenant en outre des moyens (8) pour la retenue amovible, le long de ladite direction de conjugaison (D), dudit corps tubulaire (2) vers au moins l'un d'entre ladite poche de rétention (5) et/ou ledit élément d'accès (6), dans la direction d'espacement mutuel,
ledit dispositif comprenant au moins un corps semblable à une collerette (9) associé monobloc audit corps tubulaire (2) et formé avantageusement dans une région intermédiaire entre ladite première extrémité (3) et ladite seconde extrémité (4) ; ledit corps semblable à une collerette (9) convenant pour venir en butée contre ledit élément d'accès (6), et lesdits moyens de retenue (8) étant associés audit corps semblable à une collerette (9) pour retenir de manière amovible ledit élément d'accès (6) et le corps semblable à une collerette (9) couplés ensemble,
**caractérisé en ce que** lesdits moyens de retenue (8) comprennent au moins une paire de crochets associés au corps semblable à une collerette et **en ce que** lesdits crochets sont espacés l'un de l'autre d'une distance supérieure au plus petit axe dudit rebord périmétrique elliptique, ladite distance correspondant substantiellement à l'axe principal dudit rebord périmétrique elliptique, de sorte que, après le mouvement de translation dudit dispositif (1) le long de ladite direction de conjugaison (D) dans la direction d'approche vers ladite poche de rétention (5) pour faire en sorte que ledit corps semblable à une collerette (9) vienne en butée contre ledit élément d'accès (6) par rotation dudit corps semblable à une collerette (9) par rapport audit élément d'accès (6), les crochets (10) soient amenés à interférer avec une région d'emboitement (11) dudit élément d'accès (6) afin de retenir, le long de la direction de conjugaison (D) dans la direction d'espacement mutuel, une partie (12) dudit corps tubulaire (2) à l'intérieur dudit élément d'accès (6).

2. Le dispositif (1) de la revendication 1, **caractérisé en ce que** ladite partie (12) convient pour permettre une insertion par coulissement avec ajustement étroit dans au moins une partie dudit élément d'accès (6) pour le perçage de ladite poche de rétention (5).

3. Le dispositif (1) de la revendication 1 ou 2, **caractérisé en ce que** lesdits crochets (10) sont réalisés en un matériau élastiquement accommodant, pour le couplage avec interverrouillage dudit corps tubulaire (2) et/ou dudit corps semblable à une collerette (9) avec ledit élément d'accès (6).

4. Le dispositif (1) d'une ou plusieurs des revendications précédentes, **caractérisé en ce que** ladite partie (12) présente une longueur qui est au moins substantiellement égale à la longueur dudit élément d'accès (6), ledit embout (7), pendant le perçage, étant apte à faire saillie au moins partiellement dans ladite poche de rétention (5) à proximité de l'élément d'accès (6).

5. Le dispositif (1) d'une ou plusieurs des revendications précédentes, **caractérisé en ce que** ledit corps tubulaire (2), ledit corps semblable à une collerette (9) et lesdits moyens de retenue (8) se présentent sous forme d'une pièce unique de manière à former un corps monolithique.

6. Le dispositif (1) d'une ou plusieurs des revendications précédentes, **caractérisé en ce que** ladite seconde extrémité (4) est pourvue d'au moins un connecteur de type Luer-Lok.

7. Un nécessaire pour le transport et l'administration de fluides physiologiques pour usage médical et analogues, comprenant au moins une poche (5) pour la rétention d'au moins un fluide physiologique pour usage médical pourvue d'au moins un élément d'accès (6), l'élément d'accès (6) comprenant un rebord périmétrique qui s'étend plus loin que ladite poche de rétention (5), qui est substantiellement semblable à une collerette et de forme substantiellement elliptique, et au moins un dispositif de perçage (1) selon une ou plusieurs des revendications 1 à 6.

8. Le nécessaire de la revendication 7, comprenant en outre au moins l'un d'entre un dispositif de perfusion ou d'administration et des moyens de fermeture temporaire de ladite seconde extrémité (4) pouvant être associés audit dispositif de perçage (1).
